# EUROPÄISCHE PATENTSCHRIFT

(11) **EP 2 773 400 B1**
(45) Veröffentlichungstag und Bekanntmachung des Hinweises auf die Patenterteilung: **15.03.2017**
(21) Anmeldenummer: 12797736.1
(22) Anmeldetag: 05.11.2012
(51) Int. Cl.: A61M 5/28

(54) **EINMALSPRITZE**
SINGLE-USE SYRINGE
SERINGUE À USAGE UNIQUE

(30) Priorität: 04.11.2011 DE 102011055076; 17.07.2012 DE 102012106437
(43) Veröffentlichungstag der Anmeldung: 10.09.2014
(73) Patentinhaber: F + K Innovationen GmbH & Co.KG, 76543 Baden-Baden (DE)
(72) Erfinder: Fuchs, Karl-Heinz, 78315 Radolfzell (DE); Umsonst-Kübler, Petra, 76534 Baden-Baden (DE)
(74) Vertreter: Patentanwälte und Rechtsanwalt Weiß, Arat & Partner mbB
(86) Internationale Anmeldenummer: PCT/EP2012/071776
(87) Internationale Veröffentlichungsnummer: WO 2013/064676

(56) Entgegenhaltungen:
- EP-A1- 2 368 590
- US-A- 2 551 902
- US-A- 3 045 673
- US-B1- 7 611 495

## Beschreibung

### TECHNISCHES GEBIET

Die Erfindung betrifft eine Einmalspritze nach dem Überbegriff des Anspruchs 1.

### STAND DER TECHNIK

Aus dem Stand der Technik sind verschiedene Einmalspritzen bekannt, welche aber sehr materialaufwändig sind und entsprechend viel Müll verursachen. Die bekannten Einmalspritzen werden nach dem Gebrauch als Ganzes weggeworfen und sind grundsätzlich noch verwendbar, was wiederum den Nachteil birgt, dass sie im Drogenmilieu mehrmals eingesetzt werden, was gerade die Ansteckungsgefahr von über die Blutbahn übertragbaren Krankheiten erhöht.

In diesem Zusammenhang wird auf die US 7 611 495 B1 hingewiesen, welche eine Vorrichtung zum Ausbringen einer voreinstellbaren Menge von Medium aus einem Spritzenkörper offenbart.
Daneben wird auf die US 3 045 673 A hingewiesen. Dort ist ebenfalls eine Vorrichtung zum Ausbringen einer voreinstellbaren Menge von Medium aus einem Spritzenkörper offenbart.
In der EP 2 368 590 A1 ist eine Vorrichtung mit einem knopfbetätigbaren Rastermecahnismus gezeigt, welcher auf einen Stössel einer Spritze aufbingabr ist und bei Betätigen des Knopfes den Stössel vorbestimmt bewegt. Der Vollständigkeit halber wird auf die US 2 551 902 A hingewiesen. Dort ist ein weiterer Dosiersaufsatz für eine Spritze gezeigt.

### AUFGABE DER ERFINDUNG

Aufgabe der Erfindung ist es eine Einmalspritze zur Verfügung zu stellen, welche die Nachteile aus dem Stand der Technik weitestgehend abstellt und einen benutzerfreundlichen Aufbau aufweist.

### LÖSUNG DER AUFGABE

Die Aufgabe wird durch die Merkmale des Anspruchs 1 gelöst.

Beider erfindungsgemässen Einmalspritze handelt es sich um eine Spritzenkonstruktion, welche alle über eine Spritze einbringbaren Medien in den menschlichen Körper einbringen kann.

Die Einmalspritze ist im Wesentlichen zweiteilig aufgebaut. Das heisst es ist ein Lagerelement und ein Auebringelement vorhanden.

Die beiden Teile sind trennbar miteinander gekeppelt, wobei die Kopplung derart gestaltet ist, dass das Ausbringelement von einem beispielsweise entleertes Lagerelement abnehmbar und an einem anderen Lagerelement wiederverwendbar ist oder auf dem ursprünglichen Legerelement ankoppelbar ist. In diesem Zusammenhang spielt der Inhalt des Legerelements eine untergeordnete Rolle. Regelmässig wird in das Legerelement ein irgendwie gearteter Wirkstoff eingebracht.

Daneben ist es auch denkbar, dass das Lagerelement aus unterschiedlichen Materialien wie Kunststoff edler Glas hergestellt ist.

In diesem Zusammenhang wird noch darauf hingewiesen, dass des Lagerelement oder die Ampulle einen beweglichen Stopfen am distalen Ende und eine Injektionsnadel bzw Nadel an dem proximalen Ende aufweist.

In einem erfindungsgemässen Ausführungsbeispiel ist eine Einmalspritze derart ausgeführt, dass das anzubringende Volumen des Mediums voreinstellbar ist. Dazu wird ein Zusammenspiel von einer Mutter, welche an einer Mittelachse eines Kolbens angebracht ist, genutzt. Die Mutter wird über ein Gewinde automatisch voreingestellt, wie weit der Kolben in die Aufnahme für das Lagerelement oder die Ampulle hineinragen soll. D. h. bei Betätigen des Ausbringelementes wird der Kolben nur bis zum Anschlag der Mutter an einem Anschlag betätigbar sein. Dies wiederum führt dazu, dass nur ein voreingestelltes Volumen des Mediums aus der Ampulle ausbringbar ist.

Ein weiteres Ausführungsbeispiel einer erfindungsgemässen Einmalspritze ist derart angeordnet, dass der Kolben mit dem Stopfen zusammenwirkt. Dabei greift der Kolben in die Ampulle hinein und bewegt den Stopfen in Richtung der Nadel. Weiter ist der Kolben einstückig mit einem Federelement ausgebildet, wobei das Federelement den Kolben aus seiner Aufnahme des Ausbringelementes rückstellt. Die erfindungsgemässe Einmalspritze weist weiterhin die Besonderheit auf, dass das Ausbringelement zumindest zwei Betätigungsflächen umfasst, welche jeweils endseitig der Kraftausdehnungsrichtung des Federelementes angeordnet sind und einstückig mit dem Federelement ausgebildet sind. Dadurch wird vorteilhaft erreicht, dass der Kolben durch zwei an den Betätigungsflächen anliegenden Fingern, welche gegen die Kraft des Federelementes zu einander gedrückt werden in die Ampulle hineingestossen wird. Beim Lösen des Fingergriffs, stellt das Federelement die Betätigungsflächen in ihre Ausgangsposition zurück.

Weitere Vorteile, Merkmale und Einzelheiten der Erfindung ergeben sich aus der nachfolgenden Beschreibung bevorzugter Ausführungsbeispiele sowie anhand der Zeichnungen, diese zeigen in
Figur 1 einen Querschnitt eines erfindungsgemässen Ausbringelementes in Form eines Betätigungsadapters sowie eines erfindungsgemässen Lagerelementes in Form einer Reservoireinheit in einem getrennten Zustand;
Figur 2 einen Querschnitt der Elemente der Figur 1 in einem eingebauten Zustand;
Figur 3 einen Querschnitt eines Teilbereichs einer weiteren Ausführungsform des Ausbringelementes und des Lagerelementes in einem eingebauten Zustand;
Figur 4 eine Seitenansicht eines Teilbereichs des Ausbringelementes und des Lagerelementes der Figur 3 im eingebauten Zustand.

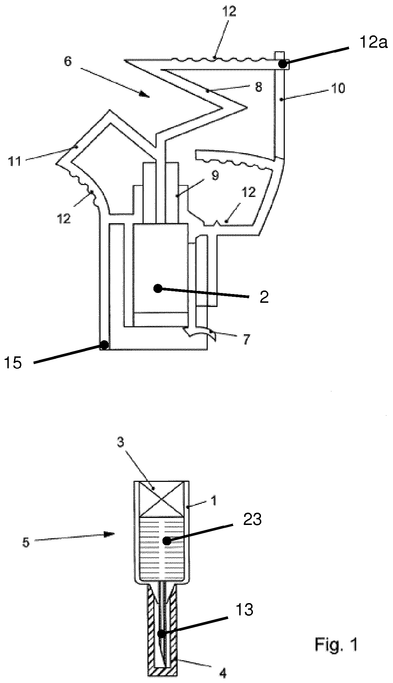

Wie in Figur 1 zu sehen weist eine erfindungsgemässe Einmalspritze ein Ausbringelement 6 und ein Lagerelement 5 auf.

Das Ausbringelement 6 ist in dieser Ausführungsform als ein Betätigungsadapter 11 geformt. Der Betätigungsadapter 11 ist in Figur 1 in einem vorgespannten Zustand abgebildet. Dem im Wesentlichen einstückig ausgebildeten Betätigungsadapter 11 sind hierbei mehrere als Griffelemente 12 ausgebildete Betätigungsflächen 12 angeformt. Weiterhin weist der Betätigungsadapter 11 einen als Federelement 8 ausgebildeten Bereich auf. Dem Betätigungsadapter 11 ist etwa mittig eine Aufnahme 2 angeformt. An einer dem Einbringen eines Gegenstandes, beispielsweise einer Ampulle 1, dienenden Öffnung ist dem die Aufnahme 2 bildenden Bereich des Betätigungsadapters 11 ein Ver- und Entriegelungsbügel 7 angeformt. Angrenzend an ein der Öffnung gegenüberliegendes Ende der Aufnahme 2 weist der Betätigungsadapter 11 einen Kolben 9 auf. Weiterhin weist der Betätigungsadapter 11 einen Anschlag 15 auf.

Das Lagerelement 5 ist in dieser Ausführungsform als eine Reservoireinheit 5 ausgebildet. Die Reservoireinheit 5 umfasst eine Ampulle 1, einen in der Ampulle 1 liegenden Stopfen 3 sowie eine Nadel 13. In dieser Ausführungsform ist die Nadel 13 von einer Schutzkappe 4 umgeben.

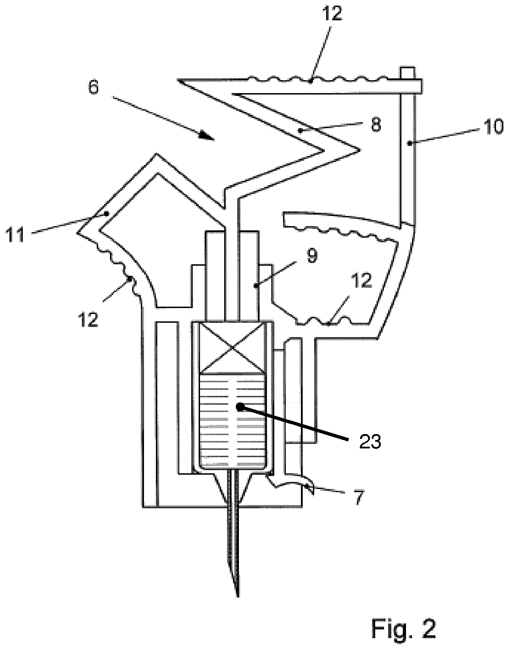

In Figur 2 ist die erfindungsgemässe Einmalspritze in eingebautem Zustand zu sehen. Es sei an dieser Stelle ausdrücklich darauf hingewiesen, dass die Positionszahlen und Bezeichnungen aus Figur 1 ebenfalls für Figur 2 gelten, auch wenn diese in Figur 2 nur teilweise aufgeführt sind.

Die Funktionsweise der in den Figuren 1 und 2 dargestellten Ausführungsformen ist folgende:
Der Betätigungsadapter 11 wird durch Einbringen des Kolbens 9 in ein dem Ver- und Entriegelungsbügel 7 gegenüberliegendes Ende der Aufnahme 2 sowie durch In-Eingriff-bringen eines Endstücks 25 des Griffelementes 12 mit einem Führungs- und Betätigungselement 10 vorgespannt. Die mit einer Wirkstofflösung 23 gefüllte Reservoireinheit 5 kann sodann von einem Benutzer in den Betätigungsadapter 11 eingebracht und mittels des Ver- und Entriegelungsbügels 7 gesichert werden. Für eine Injektion, beispielsweise eine subkutane oder eine intravenöse Injektion, kann der Benutzer den Betätigungsadapter 11 an eine Injektionsstelle ansetzen, wobei ein Anschlag 15 eine Einstichtiefe vorgeben kann. Die Injektion der Wirkstofflösung 23 durch den Benutzer erfolgt durch ein entsprechendes Drücken auf die Griffelemente 12. Dieses Drücken der Griffelemente 12 geschieht vorteilhafterweise mit Daumen, Mittel- und Zeigefinger. Dieses Drücken führt zu einer Bewegung des Kolbens 9 und somit auch des am Kolben 9 anliegenden Stopfens 3 der Reservoireinheit 5 in richtung der Nabel 13, wodurch die Wirkstofflösung 23 injiziert wird. Der Benutzer kann den Ver- und Entriegelungsbügel 7 nach der Injektion in eine Richtung von dem Eingriff 2 bzw. von der Ampulle 1 wegbewegen und anschliessend die Ampulle 1 aus dem Ausbringelement 6 entfernen. Der Betätigungsadapter 11 kann nun wiederverwendet werden.

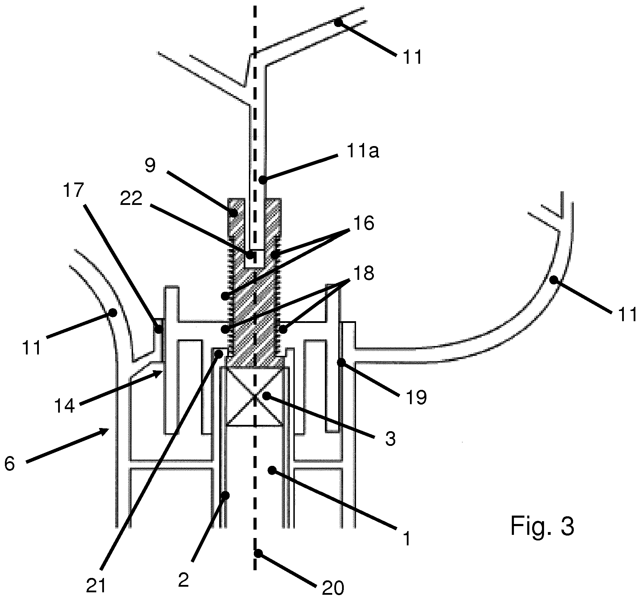

Figur 3 zeigt einen Teilbereich einer weiteren Ausführungsform der erfindungsgemässen Vorrichtung, wobei die Ampulle 1 hier mehrfach verwendet werden kann. Der Betätigungsadapter 11 weist in dieser Ausführungsform eine Aufnahme 19 auf. Weiterhin ist dem Betätigungsadapter 11 ein Vergrösserungsablesebereich 17 angeformt. Der Kolben 9 ist in dieser Ausführungsform lösbar mit dem Betätigungsadapter 11 verbunden, wobei ein als Vieleck geformtes Endstück 24 des Betätigungsadapters 11 in ein an einem Endstück des Kolbens 9 befindliches Sackloch 22 eingebracht ist, wobei dieses Sackloch 22 als Vieleck mit zum Endstück 24 korrespondierender Geometrie geformt ist. Weiterhin ist der Kolben 9 in dieser Ausführungsform mit einem Aussengewinde 16 versehen, welche mit einem Innengewinde 18 einer Mutter 14 in Eingriff gebracht werden kann. Die Mutter 14 wiederum kann in die Aufnahme 19 des Betätigungsadapters 11 eingeführt werden.

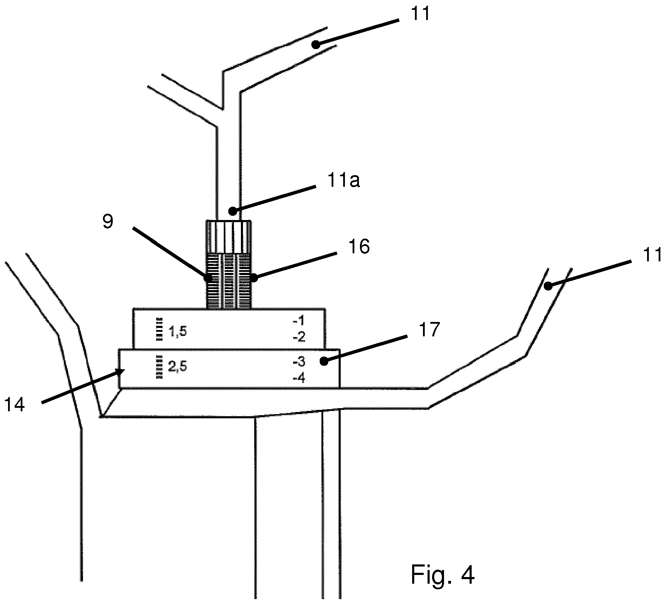

Figur 4 zeigt eine Seitenansicht des in Figur 3 dargestellten Teilbereichs der Vorrichtung. Die Mutter 14 ist aussen mit einer Skalierung versehen. Der Vergrösserungsablesebereich 17 des Betätigungsadapters 11 und die Skalierung auf der Mutter 14 sind so zueinander positioniert, dass die Skalierung vom Vergrösserungsablesebereich 17 vergrössert wird.

Die Funktionsweise der in den Figuren 3 und 4 dargestellten Ausführungsform ist folgende:
Das Endstück 24 des Betätigungsadapters 11 wird mit dem Sackloch 22 des Kolbens 9 verbunden. Der Betätigungsadapter 11 wird nach einem Einfügen des Kolbens 9 wie oben beschrieben vorgespannt, wobei in dieser Ausführungsform die Mutter 14 in eine entsprechende Aufnahme 19 eingebracht wird. Die axiale Position der Mutter 14 bezüglich der Mittelachse 20 des Kolbens 9 wird von Benutzer derart vorgenommen, dass bei der Injektion der Anschlag 21 der Mutter 14 so auf dem Betätigungsadapter 11 zu liegen kommt, dass das gewünschte Volumen an Wirkstofflösung 23 injiziert wurde. Durch die Ausformung des Endstücks 24 und des Sacklochs 22 als Vielecke, beispielsweise Sechsecke, wobei diese Vielecke die gleiche Geometrie aufweisen, wird eine unbeabsichtigte Drehung des Kolbens 9 und somit eine Veränderung eines Volumens der zu injizierenden Wirkstofflösung 23 verhindert.

**Bezugszeichenliste**

| | | | | | |
|---|---|---|---|---|---|
| 1 | Ampulle | 34 | | 67 | |
| 2 | Aufnahme | 35 | | 68 | |
| 3 | Stopfen | 36 | | 69 | |
| 4 | Schutzkappe | 37 | | 70 | |
| 5 | Reservoireinheit | 38 | | 71 | |
| 6 | Ausbringelement | 39 | | 72 | |
| 7 | Ver- und Entriegelunsbüqel | 40 | | 73 | |
| 8 | Federelement | 41 | | 74 | |
| 9 | Kolben | 42 | | 75 | |
| 10 | Führungs- und Betätigungselement | 43 | | 76 | |
| 11 | Betätigungsadapter | 44 | | 77 | |
| 12 | Betätigungsfläche | 45 | | 78 | |
| 13 | Nagel | 46 | | 79 | |
| 14 | Mutter | 47 | | | |
| 15 | Anschlag | 48 | | | |
| 16 | Aussengewinde | 49 | | | |
| 17 | Vergrösserungsablesebereich | 50 | | | |
| 18 | Innengewinde | 51 | | | |
| 19 | Aufnahme | 52 | | | |
| 20 | Achse | 53 | | | |
| 21 | Anschlag | 54 | | | |
| 22 | Sackloch | 55 | | | |
| 23 | Wirkstofflösung | 56 | | | |
| 24 | Endstück | 57 | | | |
| 25 | Endstück | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Einmalspritze zur Einbringung eines Mediums (23) in den menschlichen Körper mit einem Lagerelement (5) oder einer Ampulle (1) und einem Ausbringelement (6), wobei das Lagerelement (5) oder die Ampulle (1) lösbar mit dem Ausbringelement (6) verbunden ist
**dadurch gekennzeichnet,**
**dass** ein Kolben (9) eine drehbare Mutter (14) aufweist, wobei der Kolben (9) mit dem Stopfen (3) zusammenwirkt und der Kolben (9) einstückig mit einem Federelement (8) ausgebildet ist, wobei das Federelement (8) den Kolben (9) aus seiner Aufnahme (2) des Ausbringelementes (6) rückstellt und das Ausbringelement (6) zumindest zwei Betätigungsflächen (12) umfasst, welche jeweils endseitig der Kraftausdehnungsrichtung des Federelementes (8) angeordnet sind und einstückig mit dem Felderelement (8) ausgebildet sind, wobei ein Ver- und Entriegelungsbügel (7) in eine Richtung von der Aufnahme (2) bzw. von der Ampulle (1) wegbewegbar ist und die Ampulle (1) aus dem Ausbringelement (6) entfernbar ist, wobei das Lagerelement (5) oder die Ampulle (1) eine Nadel (13) umfasst und das Lagerelement (5)
oder die Ampulle (1) einen beweglich gelegenen Stopfen (3) umfasst, welcher geeignet ist das Medium (23) aus dem Inneren des Lagerelements (5) oder der Ampulle (1) aus dem proximalen Ende der Nadel (13) zu verbringen

2. Einmalspritze nach Anspruch 1, **dadurch gekennzeichnet, dass** das Ausbringelement (6) eine Federkonstruktion zur Betätigung eines Ausbringimpulses des Mediums aus dem Lagerelement (5) oder die Ampulle (1) aufweist.

3. Einmalspritze nach Anspruch 1 oder 2, **dadurch gekennzeichnet, dass** das Lagerelement (5) oder die Ampulle (1) in dem Ausbringelement (6) einclipsbar ist.

4. Einmalspritze nach den Ansprüchen 1 bis 3, **dadurch gekennzeichnet, dass** das auszubringende Volumen des Mediums (23) voreinstellbar ist.

## Claims

1. A disposable syringe for the introduction of a medium (23) into the human body, having a storage element (5) or an ampoule (1) and a discharge element (6), wherein the storage element (5) or the ampoule (1) is detachably connected to the discharge element (6), **characterised in that** a plunger (9) has a rotatable nut (14), wherein the plunger (9) cooperates with the stopper (3) and the plunger (9) is in one piece with a spring element (8), wherein the spring element (8) restores the plunger (9) from its receiver (2) of the discharge element (6) and the discharge element (6) comprises at least two actuating surfaces (12) which are in each case arranged at the end of the force extension direction of the spring element (8) and which are in one piece with the spring element (8), wherein a locking and unlocking clip (7) is movable in a direction away from the receiver (2) or from the ampoule (1) and the ampoule (1) is removable from the discharge element (6), wherein the storage element (5) or the ampoule (1) comprises a needle (13) and the storage element (5) or the ampoule (1) comprises a movably located stopper (3) which is suitable for bringing the medium (23) from the interior of the storage element (5) or of the ampoule (1) and from the proximal end of the needle (13)

2. A disposable syringe according to claim 1, **characterised in that** the discharge element (6) has a spring construction for actuation of a discharge impulse of the medium from the storage element (5) or the ampoule (1).

3. A disposable syringe according to claim 1 or 2, **characterised in that** the storage element (5) or the ampoule (1) can be clipped in the discharge element (6).

4. A disposable syringe according to claims 1 to 3, **characterised in that** the volume, of the medium (23,) to be discharged is presettable.

## Revendications

1. Seringue à usage unique pour l'introduction d'un milieu (23) dans le corps humain avec un élément d'appui (5) ou une ampoule (1) et un élément de distribution (6), l'élément d'appui (5) ou l'ampoule (1) étant connecté de manière amovible à l'élément de distribution (6),
**caractérisée par le fait**
**qu'**un piston (9) présente un écrou rotatif (14), le piston (9) coopérant avec le bouchon (3) et le piston (9) étant réalisé d'une seule pièce avec un élément de ressort (8), l'élément de ressort (8) rappelant le piston (9) hors de son moyen de réception (2) de l'élément de distribution (6) et l'élément de distribution (6) comportant au moins deux surfaces d'actionnement (12) qui sont disposées, chacune, du côté de l'extrémité de la direction d'expansion de force de l'élément de ressort (8) et sont réalisées d'une seule pièce avec l'élément de ressort (8), un étrier de verrouillage et déverrouillage (7) pouvant être éloigné dans une direction du récipient (2) ou de l'ampoule (1) et l'ampoule (1) pouvant être séparée de l'élément de distribution (6), l'élément d'appui (5) ou l'ampoule (1) comportant une aiguille (13) et l'élément d'appui (5) ou l'ampoule (1) comportant un bouchon situé mobile (3) qui convient pour déplacer le milieu (23) hors de l'intérieur de l'élément d'appui (5) ou de l'ampoule (1) à partir de l'extrémité proximale de l'aiguille (13).

2. Seringue à usage unique selon la revendication 1, **caractérisée par le fait que** l'élément de distribution (6) présente une structure à ressort pour actionner une impulsion de distribution du milieu à partir de l'élément d'appui (5) ou de l'ampoule (1).

3. Seringue à usage unique selon la revendication 1 ou 2, **caractérisée par le fait que** l'élément d'appui (5) ou l'ampoule (1) peut être encliqueté dans l'élément de distribution (6).

4. Seringue à usage unique selon les revendications 1 à 3, **caractérisée par le fait que** le volume de milieu (23) à distribuer est préréglable.
